# FASCICULE DE BREVET EUROPEEN

(11) **EP 1 143 895 B1**
(45) Date de publication et mention de la délivrance du brevet: **25.05.2005**
(21) Numéro de dépôt: 99941739.7
(22) Date de dépôt: 13.09.1999
(51) Int. Cl.: A61F 13/00

(54) **COMPRESSE NON ADHERENTE STERILE**
STERILE, NICHTKLEBENDE KOMPRESSE
STERILE NON-ADHESIVE COMPRESS

(30) Priorité: 18.09.1998 FR 9811676
(43) Date de publication de la demande: 17.10.2001
(60) Demande divisionnaire: 05006102.7
(73) Titulaire: Holding Urgo Participations - HUP, 21300 Chenove (FR)
(72) Inventeur: GUILLEMET, Alain, F-21121 Fontaine lès Dijon (FR); FASNE, Michel, F-21240 Talant (FR)
(74) Mandataire: Hubert, Philippe
(86) Numéro de dépôt international: PCT/FR1999/002173
(87) Numéro de publication internationale: WO 2000/016725

(56) Documents cités:
- EP-A- 0 521 761
- WO-A-89/02754
- US-A- 5 681 579

## Description

La présente invention concerne une compresse non adhérente stérile destinée à être appliquée directement au contact d'une plaie.

### Art antérieur

On connaît depuis longtemps les avantages d'une interface grasse que l'on met en contact direct avec une plaie afin de favoriser la cicatrisation, tout en assurant une séparation entre ladite plaie et une compresse absorbante. Le Tulle Gras Lumière, commercialisé par la société SOLVAY PHARMA, représente l'un des produits fréquemment utilisés pour recouvrir les plaies cutanées. Ce produit, formé d'une trame en viscose à mailles larges, enduite d'un corps gras à base de vaseline et de baume du Pérou présente cependant des inconvénients, tels que par exemple, de fréquentes adhérences à la plaie, ou la perte de corps gras sur les outils de manipulation ou sur la plaie après retrait du pansement. Il existe également d'autres produits, commercialisés ou décrits dans la littérature, susceptibles de remplir les mêmes fonctions que le Tulle gras précédemment cité. On connaît par exemple le pansement de marque JELONET commercialisé par la société Smith et Nephew, qui est une gaze de coton imprégnée de paraffine, le pansement de marque ADAPTIC (Johnson et Johnson) qui est un tricot de viscose imprégné d'une émulsion huile dans l'eau ; ces différents produits ont un comportement assez proche du Tulle Gras Lumière précédemment évoqué. On connaît aussi le produit de marque MEPITEL, commercialisé par la société MÖLNLYCKE, correspondant au brevet EP 261167, qui est un filet extensible et élastique recouvert d'un gel de silicones hydrophobe capable d'adhérer à une peau sèche ; ce produit reste cependant peu utilisé en raison du prix élevé des gels de silicones et d'un pouvoir adhésif important. Parmi les documents publiés dans un domaine proche, on peut citer EP 497 607 qui préconise l'utilisation d'une résine adhésive hydrophile sur un pansement en filet, mais qui, comme le produit précédent, présente un pouvoir adhésif important sur la peau périlésionnelle et peut entraîner de ce fait un retrait douloureux du pansement. EP 521 761 décrit un pansement cicatrisant constitué d'une couche continue d'un élastomère tribloc à séquence centrale saturée, fortement plastifié, qui forme un pansement occlusif, non adhérent et très hydrophobe. Dans un domaine proche, on peut citer aussi EP 567 704 qui décrit un pansement antiseptique formé d'un matériau hydrogel imprégné dans une couche absorbante qui gonfle en présence d'humidité ; dans ce cas, il s'agit d'une couche continue qui semble prévue essentiellement pour faire un pansement sur des plaies non exsudatives et surinfectées. Dans un domaine d'application analogue, EP 420 841 décrit un pansement, prévu pour libérer un principe actif, formé d'une bande adhésive sur laquelle sont collés des corpuscules constitués d'une matrice hydrophobe dans laquelle sont dispersées des particules hydrophiles contenant le principe actif. EP 752 840 revendique un pansement formé d'un polymère thermoplastique formant hydrogel qui est imprégné dans un substrat fibreux. Le polymère susceptible de former un hydrogel est obtenu par copolymérisation de groupements hydrophobes et de groupements hydrophiles. Le produit obtenu par la copolymérisation est imprégné dans les fils du substrat qui est constitué par exemple d'une gaze de coton. Le produit obtenu peut être considéré comme étant un polymère présentant un caractère hydrophobe et un caractère hydrophile prédominant, de sorte que l'hydrogel soit fortement absorbant. On connaît également selon EP 617938, un pansement composé d'une feuille occlusive et une couche de polymère discontinue contenant un hydrocolloïde, le but recherché étant d'obtenir une absorption supérieure et une prévention des fuites.

Ces différents produits ne donnent toutefois pas entière satisfaction, soit en raison d'un prix élevé, soit en raison de difficultés liées à la manipulation lors de la pose ou du retrait du pansement, soit encore parce que le résultat attendu, c'est-à-dire une cicatrisation régulière et rapide de la plaie, ne peut être obtenue que très difficilement.

Sur ce dernier point qui est certainement le plus important, on sait que la cicatrisation de la plaie ne peut évoluer favorablement que si le pansement n'adhère pas aux tissus nouvellement régénérés et que si les exsudats sont éliminés tout en laissant la plaie humide.

A l'usage, il apparaît que les pansements de type Tulle Gras connus présentent le plus souvent une adhésion importante à la plaie, ce qui entraîne généralement un retrait douloureux du pansement et réduit considérablement la rapidité de guérison en raison des perturbations causées par le retrait du pansement. On s'aperçoit par ailleurs que si la compresse est trop hydrophobe, la plaie a tendance à sécher, et que, si la compresse est totalement hydrophile, la couche en contact avec la plaie gonfle, obture les éventuels passages ménagés dans la couche de contact et peut provoquer une macération de la plaie.

Dans le domaine médical, il apparaît donc comme souhaitable de disposer d'une compresse de contact avec la plaie qui soit parfaitement anti-adhérente sur les tissus régénérés, et qui maintienne des conditions d'humidité optimales favorables à la cicatrisation, tout en évitant le risque de macération.

### Objet de l'invention

Selon l'invention, on propose une solution technique au problème évoqué par le moyen d'une compresse non adhérente stérile, du type comprenant un tissu flexible à mailles ouvertes, ledit tissu comprenant des fils qui sont enrobés par un gel cohésif et non adhérent de façon à laisser les mailles essentiellement non obturées qui est caractérisée en ce que le gel est formé d'une matrice élastomérique hydrophobe fortement plastifiée et contenant, en faible quantité, une dispersion de particules hydrophiles d'un hydrocolloïde.

Selon l'une des formes préférées de l'invention, le matériau constituant le tissu est une fibre synthétique à filaments longs ou continus et plus favorablement, un fil de polyester.

Selon une autre forme préférée de l'invention, la matrice hydrophobe est à base d'un élastomère tribloc de type S-EB-S à poids moléculaire élevé.

Selon encore une autre forme préférée de l'invention, l'élastomère est plastifié à l'aide d'un mélange d'huile de paraffine et de vaseline dans une proportion d'au moins 65 % en poids du gel.

Selon une autre forme préférée de l'invention, l'hydrocolloïde en dispersion dans le gel est un sel de sodium de la carboxyméthylcellulose.

### Description détaillée

On s'est aperçu en effet que l'addition d'une petite quantité d'hydrocolloïde en dispersion dans une matrice élastomérique hydrophobe, cohésive et élastique, conférait à la surface du gel obtenu un caractère hydrophile venant se combiner au caractère hydrophobe de la matrice fortement plastifiée par une huile, mais n'induisait pas de pouvoir absorbant de l'eau. Ces propriétés hydrophobe et hydrophile, comparables à un caractère amphiphile de la surface du gel qui vient au contact direct avec la plaie, se traduisent par un résultat extrêmement favorable au processus de guérison de la plaie : un degré d'humidité optimal maintenu en surface et la présence de corps gras isolant la structure du pansement entraînent une cicatrisation plus rapide et une totale non-adhérence de la compresse sur la plaie.

L'utilisation d'un gel cohésif, élastique, stable en milieu humide, permet de bien emprisonner les fils du tissu qui restent parfaitement isolés de la plaie aussi longtemps que la compresse reste en place ; on ne risque donc à aucun moment d'établir un contact direct entre le fil et les tissus régénérés, ce qui pourrait provoquer une inclusion de fil dans la cicatrice, avec pour conséquence une destruction douloureuse des tissus au retrait du pansement. L'addition d'une quantité importante de plastifiant huileux permet de conférer des propriétés d'élasticité et de flexibilité très marquées à la matrice hydrophobe ; on obtient ainsi une compresse très souple qui s'adapte bien à la surface à protéger et qui ne se détériore à aucun moment en raison d'une forte cohésion et d'une élasticité de la matrice supérieure à l'élasticité du fil enrobé. Par ailleurs, le plastifiant huileux, préférentiellement obtenu à partir d'un mélange d'huile minérale et de vaseline officinale, donne un aspect gras et des propriétés de non-adhérence à la surface de la compresse ; il s'ensuit un contact direct très limité entre l'élastomère et la plaie, l'essentiel du contact se faisant par les composés huileux, mieux tolérés que les polymères élastomériques par les tissus vivants de la plaie.

L'hydrocolloïde dispersé dans le gel en quantité relativement faible permet d'obtenir un caractère légèrement hydrophile, suffisant pour maintenir l'environnement humide favorable à la cicatrisation, mais insuffisant pour rendre le gel capable d'absorber beaucoup d'eau. En effet, ce pouvoir absorbant n'est pas souhaitable car il entraînerait un gonflement du gel qui provoquerait une obstruction progressive des ouvertures laissées dans la structure de la compresse. La compresse deviendrait alors occlusive, supprimant ainsi la possibilité d'éliminer les exsudats en entraînant un risque de macération.

Selon l'une des caractéristiques de l'invention, la compresse cicatrisante comprend un support formé d'un tissu de fils en matériau flexible et très peu extensible et non élastique. Ce support se présente sous forme d'un tissu à mailles larges ouvertes et peut être obtenu par des procédés de tissage ou de tricotage permettant de former des mailles ouvertes de taille régulière, carrées ou polygonales. Dans le cas d'un tissage, les mailles peuvent être fixées au moyen de fils de tour afin d'obtenir une bonne stabilité dimensionnelle. La dimension des mailles est telle que la surface unitaire des ouvertures est de l'ordre de 0,5 à 10 mm², préférentiellement 0,5 à 3 mm², le taux d'ouverture du tissu (rapport de la surface ouverte sur la surface totale) étant de l'ordre de 50 à 90 %. Le fil utilisé pour fabriquer le tissu est préférentiellement un fil continu à filaments, très peu extensible et non élastique, l'extensibilité ou l'allongement étant inférieure à 35 %. Par fil continu à filaments, on entend un fil formé de un ou plusieurs filaments longs retords ; le choix de filaments longs permet d'éviter les fibres courtes qui risquent de se détacher du support et venir se disperser près de la surface de contact avec la plaie. Pour la même raison, le matériau constitutif des fils est de préférence de type hydrophobe, de nature artificielle ou synthétique ; ces constituants, comme par exemple les polyesters, les polyamides, les acétates de cellulose permettent d'obtenir des filaments longs et des fils présentant beaucoup moins de fibrilles que les fils obtenus à partir de fibres courtes par exemple. Le choix de certains matériaux synthétiques tels que des polyesters donne également la possibilité de thermofixer la structure à mailles larges du support. Le tissu à mailles larges est préférentiellement réalisé avec des fils de même nature, mais on peut utiliser aussi des tissus fabriqués par exemple avec des fils de chaîne et des fils de trame qui seraient de nature différente. Enfin, un autre avantage des matériaux très peu extensibles et non élastiques, tels que les polyesters, est une mise en oeuvre plus facile pendant le procédé de recouvrement des fils du tissu par le gel.

Selon une autre caractéristique de l'invention, le support en tissu est enrobé d'un gel non adhérent d'aspect gras de façon à laisser libre une majorité des ouvertures dudit support ; ce gel est composé d'une matrice élastomérique thermoplastique tribloc hydrophobe fortement plastifiée à l'aide d'une huile ou d'un corps gras non miscible à l'eau, et contient une dispersion de particules hydrocolloïdes. Cette association d'une matrice flexible élastique hydrophobe et de particules hydrocolloïdes hydrophiles en dispersion confère au pansement des propriétés extrêmement favorables à la cicatrisation : la matrice hydrophobe donne une bonne stabilité physique à la compresse qui peut rester en place sur la plaie plusieurs jours, sans migrer ou se disperser et sans adhérer aux tissus fraîchement régénérés, et la composante hydrophile permet de maintenir un degré d'humidité favorable au processus de cicatrisation, en évitant un dessèchement de la plaie et, en conséquence, soit la formation d'une croûte, soit une adhérence du pansement à la plaie.

De façon pratique, on choisit un élastomère thermoplastique de synthèse hydrophobe de type tribloc, S-EB-S ou S-EP-S, c'est-à-dire formé par la copolymérisation de blocs de type polystyrène avec des blocs polyoléfine du type polyéthylène-butylène ou polyéthylène-propylène. On choisit, pour obtenir une matière hydrophobe conforme à l'invention, des élastomères tribloc de type S-EB-S ou S-EP-S ayant un poids moléculaire moyen ou élevé, et une viscosité Brookfield au moins égale à 300 cp. (mesure faite à 25°C, pour une solution à 10 % dans le toluène).

Le choix particulier de ce type d'élastomère, associé à un plastifiant huileux permet d'obtenir un gel fortement cohésif et élastique, d'aspect gras et ne présentant pratiquement aucun pouvoir adhésif (le pouvoir adhésif, mesuré sur une plaque de verre, est inférieur à 8g/5cm).

L'élastomère thermoplastique hydrophobe doit être plastifié par addition d'un élément huileux hydrophobe : on choisit pour cela une huile minérale ou végétale présentant à la fois une bonne compatibilité avec les élastomères précédemment décrits et une tolérance reconnue vis-à-vis des tissus de la peau. On utilise préférentiellement des huiles de paraffine de faible viscosité à base de composés paraffiniques et naphténiques ou des mélanges d'huile de paraffine et de vaseline officinale.

Parmi les produits convenant bien pour plastifier l'élastomère, on peut citer par exemple les huiles de paraffine commercialisées sous la marque ONDINA par la société SHELL, plus particulièrement l'huile commercialisée sous la référence ONDINA 15, qui, en association avec une vaseline conforme à la pharmacopée française, permet d'obtenir l'une des formulations préférées de l'invention. L'association de l'élastomère de type tribloc avec une huile plastifiante doit être faite dans des proportions telles que l'on obtienne un gel hydrophobe extensible et élastique présentant un allongement à la rupture d'au moins 200 %, avec un retour élastique d'au moins 50 %. D'une façon générale, on peut obtenir un gel suffisamment cohésif, très souple et élastique en plastifiant 100 parts d'élastomère avec 1000 à 2000 parts d'huile à faible viscosité et 0 à 400 parts de vaseline. Un mode de réalisation préféré correspond à l'association de 100 parts d'élastomère S-EB-S à poids moléculaire élevé, comme par exemple le Kraton G 1651 commercialisé par la société SHELL avec 1600 parts d'un plastifiant huileux hydrophobe composé de 95 % d'huile de paraffine à faible viscosité et dépourvue de dérivés aromatiques, et 5 % de vaseline conforme à la pharmacopée française. Cet exemple est donné à titre indicatif, et un gel conforme peut bien sûr être obtenu en mettant en oeuvre des proportions différentes et des matières différentes.

Comme indiqué précédemment, on ajoute des particules fines hydrophiles d'un hydrocolloïde en dispersion dans la matrice hydrophobe décrite ci-dessus. Par hydrocolloïde, on entend ici les composés connus de l'homme de l'art aptes à absorber de l'eau ; parmi ceux-ci, on peut citer essentiellement la pectine, les alginates et la carboxyméthylcellulose, cette dernière étant, sous forme de sel de sodium, préférée pour réaliser l'invention. L'hydrocolloïde doit être sous forme solide et finement divisé, par exemple sous forme d'une poudre dont la granulométrie moyenne est inférieure à 100 µm et mieux, inférieure à 50 µm. La quantité d'hydrocolloïde entrant dans la formulation du gel dépend du type d'hydrocolloïde employé. Il est remarquable cependant qu'une faible quantité de ce composé apporte au gel un caractère hydrophile suffisant pour maintenir un environnement humide favorable à la cicatrisation, en empêchant un dessèchement de la plaie qui pourrait conduire à une adhérence du pansement. Dans le cas de l'utilisation de carboxyméthylcellulose sodique (CMC) l'addition de 2 à 3 % seulement de CMC permet d'obtenir un gel présentant un état de surface restant légèrement humide et glissant en présence d'une plaie. Des quantités supérieures à 20 % perturbent le caractère cohésif du gel, accentuent exagérément le caractère hydrophile et ne permettent pas d'améliorer le résultat attendu tout en augmentant le risque d'un gonflement de la matrice et, par suite, d'obturation des ouvertures de la compresse. En pratique, l'hydrocolloïde, sous forme d'une poudre fine, est intégré à raison de 3 à 20 % (en masse, par rapport à la masse de la matrice hydrophobe) dans la matrice hydrophobe pendant la phase de malaxage à haute température et se trouve ainsi dispersé de façon homogène dans le gel. Le mélange intime de l'élastomère plastifié hydrophobe formant une matrice dans laquelle est dispersé l'hydrocolloïde permet d'obtenir les propriétés caractéristiques du gel amphiphile, capable de maintenir un environnement humide suffisant sans avoir le caractère absorbant des mélanges fortement chargés en hydrocolloïdes. Selon une variante du procédé, le gel hydrophobe peut être formulé de façon indépendante sans hydrocolloïde, enduit sur le support de façon à enrober les fils et laisser les ouvertures de mailles non obturées et, alors que le gel est encore chaud, on projette les fines particules d'hydrocolloïde sur la surface du gel. Selon cette variante du procédé, une quantité de l'ordre de 0,2 à 0,5 % en poids d'hydrocolloïde est suffisante pour obtenir une compresse non adhérente possédant un caractère amphiphile en surface et montrant un comportement satisfaisant sur une plaie humide. Ce procédé permet également de réaliser une compresse asymétrique en ne projetant les particules d'hydrocolloïde que sur la face destinée à être exposée au contact direct de la plaie : on obtient de cette façon une compresse ayant une face hydrophile et une face hydrophobe.

De façon pratique et classique, la composition comprend également un ou plusieurs antioxydants ou stabilisants tels que par exemple des composés phénoliques vendus sous la marque IRGANOX par la société CIBA GEIGY.

Il est également possible d'ajouter des principes actifs à la formulation du gel si l'on souhaite ajouter un effet thérapeutique particulier aux propriétés cicatrisantes de la compresse. On peut pour cela disperser dans le gel des composés ayant des propriétés antiseptiques comme par exemple la sulfadiazine argentique, des antibiotiques comme par exemple la néomycine ou la polymyxine, des anti-inflammatoires non-stéroïdiens ou stéroïdiens comme par exemple l'acétonide de triamcinolone.

Selon l'un des modes préférés de réalisation de l'invention, on enduit d'un gel un tissu à mailles larges de façon à enrober les fils du tissu en laissant une majorité des mailles non obstruée.

Comme indiqué précédemment, on utilise un support tissé ou tricoté présentant des mailles larges rectangulaires, carrées ou polygonales dont l'ouverture correspond sensiblement à 4 à 20 mailles par cm, le tissu ayant un taux d'ouverture (rapport des surfaces ouvertes sur la surface totale) de 50 à 90 %. Le fil utilisé pour obtenir le support est de préférence un fil continu à filaments, et on choisit, pour réaliser les exemples préférés de l'invention, des fils en matériau artificiel ou synthétique, à caractère hydrophobe et d'extensibilité inférieure à 35 %. La nature du fil est, par exemple, un polyester de type polyéthylènetéréphtalate, un polyamide ou un acétate de cellulose ; on utilise de préférence un tissu à mailles larges thermofixées en fils continus de polyester (Tergal ou polyéthylènetéréphtalate), par exemple des tissus commercialisés sous le nom de marquisette, grammant environ 30 à 80 g/m². Ces tissus pratiquement non extensibles dans. les directions chaîne ou trame présentent l'avantage de se travailler plus facilement que les tissus élastiques et on obtient un enrobage plus régulier des fils.

Le gel non adhérent est obtenu préférentiellement par malaxage à chaud sans solvant (procédé dit hot-mélt), en mélangeant l'élastomère avec le plastifiant huileux, les antioxydants, et puis en ajoutant l'hydrocolloïde en poudre finement divisée. Si des principes actifs sont prévus, ceux-ci sont ajoutés en dernier.

Le procédé d'enduction du tissu par le gel doit permettre de bien emprisonner les fils dans le gel, tout en laissant une majorité d'ouvertures non obturées par le gel. Selon la structure du support utilisé, la quantité de gel utilisée sera d'environ 50 à 300 g/m², et préférentiellement 60 à 160 g/m². Compte-tenu des composants du gel, l'enduction est faite à chaud, sans solvant, selon un procédé continu dans lequel la bande de .tissu est dirigée sur un premier cylindre d'enduction recouvert d'une couche de gel en fusion d'épaisseur prédéterminée par un racle, puis sur un second cylindre qui élimine le gel compris dans les ouvertures des mailles. La bande ainsi recouverte de gel uniquement au niveau des fils est ensuite refroidie dans une zone de ventilation ascendante afin que le gel ne coule pas et reste réparti régulièrement autour des fils. Si nécessaire, on prévoit un système à jet d'air laminaire qui peut à la fois corriger la répartition du gel autour des fils et déboucher les ouvertures de mailles qui n'auraient pas été ouvertes à l'étape précédente du procédé.

Selon une variante de ce procédé, on fait passer la bande de tissu dans un bain de gel fondu à 140-150°C ; la bande recouverte de gel fondu est ensuite passée entre deux cylindres fixes pressés l'un contre l'autre avec un écartement prédéterminé de façon à éliminer l'excèdent de gel. La quantité de gel restant sur les fils dépend essentiellement de l'écartement imposé entre les cylindres fixes. La bande recouverte est ensuite refroidie et traitée de façon analogue au procédé précédent.

La bande de compresse non adhérente refroidie est ensuite recouverte de deux films de protection, par exemple des films fins en polyester. En raison du caractère non adhérent de la bande de compresse, ces films ne nécessitent pas de traitement antiadhérent et n'ont pour fonction que de faciliter l'extraction de l'emballage principal et la manipulation lors de la mise en place sur la plaie. La bande est ensuite découpée en compresses individuelles suivant des dimensions adaptées à l'utilisation, conditionnée en sachets étanches et stérilisée.

La compresse non adhérente selon l'invention peut être utilisée de façon analogue aux interfaces connues actuellement telles que par exemple le Tulle Gras Lumière. De façon classique, la compresse est placée en contact direct avec la plaie et peut être utilisée en simple ou multiple couches : la souplesse du support et du gel permet de bien appliquer la compresse sur toute la surface de la plaie, en débordant sur le pourtour jusqu'à la peau saine. La compresse stérile est ensuite recouverte d'un tampon absorbant si la plaie est fortement exsudative et l'ensemble est maintenu en place par une bande ou un ruban adhésif qui vient se fixer sur les zones périphériques de peau saine éloignées de la plaie. Le pansement ainsi réalisé peut rester en place de façon prolongée : en effet, le gel hautement cohésif ne se désintègre pas et la présence d'une quantité faible d'hydrocolloïdes maintient en surface de la plaie un degré d'humidité suffisant pour empêcher celle-ci de sécher. De plus, en raison du caractère non adhérent du gel utilisé, on peut, pratiquement sans risque, retirer le tampon absorbant qui n'adhère pas au gel sans déplacer la compresse stérile, pour contrôler l'évolution de la plaie. Bien que la compresse soit translucide, ce qui autorise l'examen de la plaie par transparence, il peut être nécessaire de retirer également cette compresse pour effectuer un contrôle visuel plus précis ou pour procéder à un traitement médicamenteux direct de la zone en cours de cicatrisation : ce retrait se fait facilement sans douleur et sans endommager les tissus nouvellement régénérés car le gel n'adhère ni à la surface de la plaie, ni à la peau périlésionnelle. De plus, en raison d'une forte cohésion du gel dans lequel les fils du tissu sont emprisonnés et la présence de CMC qui maintient un milieu légèrement humide, le retrait de la compresse peut se faire de façon intégrale, sans laisser de particules ou de corps gras comme cela se produit avec certains produits actuellement commercialisés. Le nettoyage de la plaie se trouve par conséquent nettement facilité. Avec tous ces avantages que sont une excellente cohésion associée à un caractère non adhérent sur surface humide et sur peau sèche, on réunit les meilleures conditions favorables au processus de cicatrisation de la plaie. Les exemples de réalisation suivants permettent de mieux apprécier la portée de l'invention, mais ne doivent pas être considérés comme limitatifs.

### Exemple 1

On prépare le gel par malaxage à 150°C de 8 kg d'huile de paraffine (ONDINA 15 commercialisée par la société SHELL), 1 kg (soit environ 5 % de masse totale du gel) d'élastomère S-EB-S à haut poids moléculaire (KRATON G 1651 commercialisé par la société SHELL) et 25 g d'antioxydant (IRGANOX 1010). Quand le mélange est homogène, on ajoute 1 kg (soit environ 5 % de la masse du gel) de vaseline (qualité conforme à la pharmacopée française ou Codex) et 7,2 kg (soit au total 75 % environ de la masse du gel) d'huile ONDINA 15. Après 30 minutes de malaxage la température est abaissée à 130-135°C et on ajoute 3,04 kg (soit 15 % environ de la masse de gel) de carboxyméthylcellulose sodique (ref. 7H4XF commercialisée par Aqualon). Après 40 minutes de mélange, le gel peut être utilisé pour enrober les fils du tissu. Le tissu utilisé est une marquisette thermofixée en fils de polyester (polyéthylènetéréphtalate), 33 décitex en chaîne et en trame, présentant des mailles carrées dont l'ouverture est environ 0,8 à 1 mm² ; le grammage du tissu est environ 45 g/m² (ce tissu est fabriqué par la société TEXINOV). Le tissu est enrobé d'une couche de gel par passage dans un bain de gel fondu à 135-145°C, puis l'excédent est éliminé par passage entre deux cylindres fixes dont l'écart est prédéterminé en fonction du résultat recherché. La compresse en bande est ensuite refroidie par un courant d'air froid ascendant. La quantité de gel déposée sur les fils du tissu est environ 130 g/m². La bande refroidie est complexée avec un film protecteur en polyester d'épaisseur 23 µm sur chacune de ses faces, puis découpée en feuilles pour former des compresses, chacune étant conditionnée dans une pochette étanche et stérilisée sous rayonnement β.

### Exemple 2

On utilise un tissu et un gel identiques à l'exemple 1, mais les machines sont réglées pour obtenir une bande comprenant 60 g de gel par m².

### Exemple 3

On réalise une compresse stérile analogue à l'exemple 1, à l'exception de l'hydrocolloïde qui, au lieu d'être la carboxyméthylcellulose sodique est un alginate de propylène glycol commercialisé sous la référence PROTANAL ester SDLB par la société PRONOVA BIOPOLYMER.

### Exemple 4

On fabrique une compresse de façon analogue à l'exemple 1, à l'exception des quantités de plastifiant qui sont respectivement de 13,2 kg d'huile de paraffine ONDINA 15 (soit environ 65 % de la masse totale du gel) et 3,04 kg de vaseline codex A (soit environ 15 % de la masse du gel).

### Exemple 5

On prépare une compresse de façon analogue à l'exemple 1, à l'exception de l'élastomère qui est ici un mélange de 600 g de Kraton G 1651 et 400 g de Kraton G 1652.

### Exemple 6

La compresse est préparée à partir d'un gel de formulation analogue à celui décrit dans l'exemple 1 mais le tissu utilisé est une gaze de viscose à mailles rectangulaires commercialisée par la société MOLYPHARM sous la référence 548. La quantité de gel déposée sur les fils du tissu est d'environ 180 g/m².

### Exemple 7

La compresse est fabriquée de façon analogue à l'exemple 1, à l'exception de l'élastomère qui est ici un élastomère de type S-EP-S à haut poids moléculaire de référence SEPTON 4055 obtenu auprès de la société KURARAY. Les quantités mises en oeuvre sont les mêmes que dans l'exemple 1.

### Exemple 8

La compresse est réalisée de façon analogue à l'exemple 5, à l'exception du gel élastomérique auquel on ajoute, en fin de malaxage, 200 g de sulfadiazine argentique en poudre finement divisée. Ce principe actif apporte des propriétés antiseptiques à la compresse.

Le pouvoir cicatrisant et la commodité d'utilisation des compresses de pansement selon l'invention ont été évalués et comparés à des produits existants et couramment utilisés pour protéger et soigner des plaies. L'étude a été faite sur plaie dermo-épidermique chez le cobaye.

Pour conduire ces essais, on a réalisé une plaie dermoépidermique carrée de 9 cm² sur le flanc de chaque cobaye (5 animaux par groupe) en laissant en place le muscle peaucier. Les compresses stériles à étudier sont appliquées sur la plaie et recouvertes par une gaze stérile et une bande adhésive. Le pansement est renouvelé tous les deux jours en début de traitement, puis tous les trois jours. L'évolution de la cicatrisation est ainsi suivie pendant 22 jours. L'efficacité de chacune des compresses est évaluée en suivant, selon une grille de critères établie préalablement, l'humidité du pansement, son adhérence à la plaie, le caractère inflammatoire ou hémorragique et le degré de cicatrisation de la plaie.

L'étude a été conduite comparativement sur trois exemples réalisés selon l'invention et deux produits disponibles dans le commerce. Les résultats sont les suivants :
■ avec la compresse selon l'exemple 1 précédemment décrit, une plaie est totalement cicatrisée après 22 jours, trois plaies sont presque cicatrisées et la dernière plaie présente une surface faible (0,1 cm²) non cicatrisée. Aucune adhérence du pansement n'a été observée tout au long du traitement, aucune inclusion n'a été relevée et le pansement se dessèche légèrement.
■ avec la compresse selon l'exemple 2, une plaie est totalement cicatrisée après 19 jours, trois plaies sont cicatrisées après 22 jours et la dernière plaie présente une surface faible (0,5 cm²) non cicatrisée. Aucune adhérence du pansement et aucune inclusion n'ont été observées pendant toute la durée du traitement. Le pansement reste bien humide en début de traitement et se dessèche un peu en fin de traitement.
■ avec la compresse selon l'exemple 6, aucune plaie n'est cicatrisée après 22 jours, l'une est très proche de la cicatrisation, les autres présentent une surface faible (0,5 à 0,6 cm²) non cicatrisée. Le pansement présente de très rares cas d'adhérence faible à la plaie, mais aucune inclusion n'est apparue. Le pansement se dessèche légèrement à tous les stades de la cicatrisation.
■ Le premier produit de comparaison est un tulle gras du commerce, composé d'un tissu de viscose enduit de vaseline et contenant environ 1 % de baume du Pérou (selon la description figurant au dictionnaire Vidal 1998), avec lequel deux plaies sont cicatrisées après 22 jours de traitement et les trois autres plaies sont de taille moyenne (0,6 à 1 cm²). Avec ce produit, on relève une adhérence systématique à la plaie, notamment pendant les 15 premiers jours, ainsi que des inclusions pendant les 6 premiers jours. Le pansement se dessèche nettement tout au long du traitement.
■ Le second produit de comparaison est une compresse du commerce formé d'un tricot de viscose imprégné d'une émulsion huile dans eau (selon la monographie figurant au dictionnaire Vidal 1998) ; avec cette interface, aucune plaie n'est cicatrisée après 22 jours, deux sont en cours de cicatrisation avancée (la plaie résiduelle représente 0,1 à 0,3 cm³) et deux sont encore grandes (environ 1 cm²). On note des adhérences assez fréquentes du pansement pendant les 15 premiers jours, mais très peu de cas d'inclusions. Le pansement présente un aspect sec tout au long du traitement.

A la lumière de ces différents essais, il apparaît que les compresses stériles selon l'invention, notamment lorsque celles-ci intègrent un tissu en fibres synthétiques hydrophobes, permettent de faire cicatriser les plaies plus rapidement. De plus, en raison de la non-adhérence du pansement à la plaie et à la peau périlésionnelle, elles permettent en toute circonstance un changement de pansement non douloureux et bien plus confortable pour le patient.

## Revendications

1. Compresse non adhérente stérile, destinée à être appliquée directement au contact d'une plaie, comprenant un tissu flexible à mailles ouvertes, ledit tissu comprenant des fils hydrophobes à filaments continus peu extensibles enrobés par un gel cohésif et non adhérent, ledit gel étant formé d'une matrice élastomérique hydrophobe plastifiée, **caractérisée en ce que** lesdits fils du tissu sont enrobés par ledit gel de façon à laisser les mailles essentiellement non obturées et **en ce que** ladite matrice hydrophobe comprend 100 parties d'un élastomère tribloc pour 1000 à 2000 parties d'un plastifiant huileux et 0 à 400 parties de vaseline officinale, et contient en dispersion des particules hydrophiles d'un hydrocolloïde à raison de 3 à 20 % du poids du gel.

2. Compresse pour pansement selon la revendication 1, **caractérisée en ce que** le tissu est formé de fils à filaments continus peu extensibles et non élastiques.

3. Compresse selon les revendications 1 ou 2, **caractérisée en ce que** les fils constituant le tissu sont en polyester, notamment du polyéthylènetéréphtalate.

4. Compresse selon l'une quelconque des revendications 1 à 3, **caractérisée en ce que** ledit élastomère tribloc est de type S-EB-S ou S-EP-S.

5. Compresse selon la revendication 4, **caractérisée en ce que** ledit élastomère tribloc est à haut poids moléculaire.

6. Compresse selon l'une quelconque des revendications 1 à 5, **caractérisée en ce que** ledit plastifiant huileux est à base d'huile de paraffine.

7. Compresse selon la revendication 1, **caractérisée en ce que** l'hydrocolloïde est la carboxyméthylcellulose sodique.

8. Compresse selon la revendication 1, **caractérisée en ce que** l'hydrocolloïde est un alginate de propylèneglycol.

9. Compresse selon l'une quelconque des revendications précédentes, **caractérisée en ce que** le gel contient en outre un principe actif.

10. Compresse selon la revendication 9, **caractérisée en ce que** le principe actif est un antiseptique, un antibiotique ou un anti-inflammatoire.

## Patentansprüche

1. Nicht klebende sterile Kompresse, die zum direkten Aufbringen im Kontakt mit einer Wunde ausgelegt ist, umfassend ein flexibles, offenmaschiges Gewebe, wobei das Gewebe hydrophobe Fasern aus wenig dehnbaren Endlosfilamenten, die mit einem kohäsiven und nicht haftenden Gel umhüllt sind, umfasst, wobei das Gel aus einer elastomeren hydrophoben plastifizierten Matrix geformt ist, **dadurch gekennzeichnet, dass** das Gewebe von dem Gel so eingehüllt ist, dass die Maschen im Wesentlichen nicht verstopft sind und dadurch, dass die hydrophobe Matrix 100 Teile eines Dreiblockelastomers auf 1000 bis 2000 Teile eines öligen Weichmachers und 0 bis 400 Teile offizielle Vaseline umfasst und in Dispersion hydrophile Teilchen eines Hydrokolloids in einem Verhältnis von 3 bis 20 Gew.-% des Gels enthält.

2. Verbandskompresse nach Anspruch 1, **dadurch gekennzeichnet, dass** das Gewebe aus Fasern aus wenig dehnbaren und nicht elastischen Endlosfilamenten gebildet ist.

3. Kompresse nach den Ansprüchen 1 oder 2, **dadurch gekennzeichnet, dass** die Fasern, die das Gewebe aufbauen, aus Polyester, insbesondere Polyethylenterephthalat, sind.

4. Kompresse nach einem der Ansprüche 1 bis 3, **dadurch gekennzeichnet, dass** das Dreiblockelastomer vom Typ S-EB-S oder S-EP-S ist.

5. Kompresse nach Anspruch 4, **dadurch gekennzeichnet, dass** das Dreiblockelastomer ein hohes Molekulargewicht besitzt.

6. Kompresse nach einem der Ansprüche 1 bis 5, **dadurch gekennzeichnet, dass** der ölige Weichmacher auf Paraffinölbasis ist.

7. Kompresse nach Anspruch 1, **dadurch gekennzeichnet, dass** das Hydrokolloid Natriumcarboxymethylcellulose ist.

8. Kompresse nach Anspruch 1, **dadurch gekennzeichnet, dass** das Hydrokolloid Propylenglycolalginat ist.

9. Kompresse nach einem der vorhergehenden Ansprüche, **dadurch gekennzeichnet, dass** das Gel außerdem einen Wirkstoff enthält.

10. Kompresse nach Anspruch 9, **dadurch gekennzeichnet, dass** der Wirkstoff ein Antiseptikum, ein Antibiotikum oder ein Entzündungshemmer ist.

## Claims

1. A sterile non-adhesive compress, intended to be applied directly in contact with a wound, comprising an open-meshed flexible fabric, said fabric comprising hydrophobic yarns with continuous filaments of low extensibility which are coated with a cohesive but non-adhesive gel, said gel being formed by a hydrophobic plasticized elastomeric matrix, **characterized in that** said fabric is coated by said gel so as to leave the meshes substantially unobstructed and **in that** said hydrophobic matrix comprises 100 parts of a triblock elastomer per 1000 to 2000 parts of an oily plasticizer and 0 to 400 parts of officinal vaseline and contains a dispersion of hydrophilic particles of a hydrocolloid in an amount of 3 to 20% by weight of the gel.

2. A dressing compress as claimed in claim 1, **characterized in that** the fabric is formed by nonelastic continuous multifilament yarns of low extensibility.

3. The compress as claimed in claim 1 or 2, **characterized in that** the yarns making up the fabric are made of polyester, especially polyethylene terephthalate.

4. The compress as claimed in any one of claims 1 to 3, **characterized in that** said triblock elastomer is of the S-EB-S or S-EP-S type.

5. The compress as claimed in claim 4, **characterized in that** said triblock elastomer is of high molecular weight.

6. The compress as claimed in any one of claims 1 to 5, **characterized in that** said oily plasticizer is based on paraffin oil.

7. The compress as claimed in claim 1, **characterized in that** the hydrocolloid is sodium carboxymethylcellulose.

8. The compress as claimed in claim 1, **characterized in that** the hydrocolloid is a propylene glycol alginate.

9. The compress as claimed in any one of the preceding claims, **characterized in that** the gel furthermore contains an active substance.

10. The compress as claimed in claim 9, **characterized in that** the active substance is an antiseptic, an antibiotic or an anti-inflammatory.
